# EUROPEAN PATENT SPECIFICATION

(11) **EP 3 967 243 B1**
(45) Date of publication and mention of the grant of the patent: **17.01.2024**
(21) Application number: 21196539.7
(22) Date of filing: 14.09.2021
(51) Int. Cl.: A61B 10/06, A61B 10/04

(54) **BIOPSY FORCEPS AND JAW ASSEMBLY THEREOF**
BIOPSIEZANGE UND BACKENANORDNUNG DAVON
PINCE DE BIOPSIE ET SON ENSEMBLE MÂCHOIRE

(30) Priority: 15.09.2020 CN 202010965186; 15.09.2020 CN 202022011344 U
(43) Date of publication of application: 16.03.2022
(73) Proprietor: Beijing Longmiao Medical Device Co., Ltd., 101402 Beijing Beijing (CN)
(72) Inventor: LING, Yu, Beijing, 101402 (CN); CHUN, Yu, Beijing, 101402 (CN)
(74) Representative: Wang, Bo

(56) References cited:
- US-A- 5 238 002
- US-A1- 2009 112 230
- US-B1- 6 425 910

## Description

### TECHNICAL FIELD

The present disclosure relates to a biopsy forceps and a jaw assembly thereof, and belongs to the field of medical instruments.

### BACKGROUND

In recent years, endoscopy has been widely used as an auxiliary diagnostic method in clinical medical examination. When a lesion in a patient is examined, biopsy forceps may stretch into the body through an endoscope forceps channel to clamp pathological tissues. Endoscopy is a popular diagnostic method because the patient is minimally invaded and less painful by endoscopic sampling.

With the development of a medical technology, transnasal endoscopy and tracheoscopy have been developed at present, which are more acceptable to a human body. In order to fit a small-sized lumen, the size of the biopsy forceps needs to be reduced. When the size of the biopsy forceps is reduced, higher requirements are put forward on the structural strength and assembly of the biopsy forceps. However, existing four-linkage type biopsy forceps have the disadvantages of many parts, complex structure, and large size. Directly reducing the size cannot guarantee the structural strength of some of the parts. Moreover, due to the limitation of a product size and the strength of a powder metallurgy injection molding material, the size of a hole in the middle of a handle is too small, so that the fracture problem of the hole in the middle of the handle frequently occurs, and the phenomena of unstable reliability, etc. are easily caused.

US5238002 discloses a jaw assembly according to the preamble of claim 1. US6425910 discloses another prior art jaw assembly.

### SUMMARY

The primary technical problem to be solved by the present disclosure is to provide a jaw assembly of biopsy forceps.

Another technical problem to be solved by the present disclosure is to provide biopsy forceps.

In order to achieve the above objects, the present disclosure adopts the following technical solution.

According to a first aspect of the present invention, a jaw assembly of biopsy forceps according to claim 1 is provided. The jaw assembly of biopsy forceps, comprising: a jaw base, a first jaw, a second jaw, a limiting element, a pulling member, and a shaft member, wherein
the jaw base comprises a hollow tube, a first arm, and a second arm, which are integrally formed, the first arm and the second arm standing oppositely at a front end of the hollow tube, and an elongated slot between the first arm and the second arm for accommodating the first jaw and the second jaw;
the first jaw and the second jaw are openable and closeable, and are movably arranged in the elongated slot of the jaw base;
a rear end of the first jaw and a rear end of the second jaw are relatively rotatably connected with a front end of the pulling member, and the pulling member extends rearward within a cavity of the hollow tube;
the limiting element is arranged between the first jaw and the second jaw, two ends of the limiting element are respectively fixed to the first arm and the second arm, and the limiting element is configured to limit a maximum stroke of the pulling member pushing forward; and
the first jaw and the second jaw are individually provided with a ramp for being matched with the limiting element to open or close the first jaw and the second jaw.

Preferably, a distance between the ramp of the first jaw and the ramp of the second jaw are gradually reduced to the rear end of the first jaw and the rear end of the second jaw.

Preferably, the ramp of the first jaw or the second jaw is located in front of the shaft member.

Preferably, a minimum distance between the ramp of the first jaw and the ramp of the second jaw is between the limiting element and the shaft member.

Preferably, the minimum distance is smaller than a size of the limiting element located between the ramp of the first jaw and the ramp of the second jaw.

Preferably, the first arm is provided with a first fixing slot, the second arm is provided with a second fixing slot, both ends of the limiting element are fixed to the first fixing slot and the second fixing slot respectively, and the first fixing slot and the second fixing slot are less than the elongated slot in depth.

Preferably, the first jaw and the second jaw individually comprise a jaw spoon and a handle, which are integrally formed, the handle comprises a handle body and a connecting portion, and the handle body is configured to connect the jaw spoon and the connecting portion; and
the first jaw and the second jaw are relatively rotatably connected at the connecting portion.

Preferably, the jaw spoon, the handle, and the connecting portion are integrally formed along a straight line to form a rigid structure.

Preferably, the connecting portion connects the first jaw and the second jaw and is connected to the pulling member, and the connecting portion and the pulling member together move forward or rearward relative to the jaw base.

The jaw assembly according to the invention further comprises:
a sampling needle, wherein a rear end of the sampling needle is fixed to the limiting element.

Preferably, the rear end of the first jaw and the rear end of the second jaw have opposite portions in inclined contour.

According to the invention, the pulling member comprises a pull rod and a connecting tube, a front end of the pull rod is provided with an annular connecting portion, and the pull rod is inserted into a front end of the connecting tube to be fixed.

According to a second aspect of the present disclosure, a biopsy forceps according to claim 11 is provided. The
biopsy forceps comprising the jaw assembly and further comprising: a spring tube, a controlling wire, and an operating handle, wherein a front end of the spring tube is fixed to a rear end of the jaw base, a rear end of the spring tube is fixed to the operating handle, a rear end of the pulling member is fixed to a front end of the controlling wire, the controlling wire extend rearward through the spring tube, and a rear end of the controlling wire is fixed to the operating handle.

In the biopsy forceps provided by embodiments of the present disclosure, the jaw assembly is improved, a four-linkage structure is removed from the jaw assembly, the rear ends of the first jaw and the second jaw are pushed and pulled only through the pulling member, and the first jaw and the second jaw are opened and closed in combination with the limiting effects of the limiting element and the jaw base. The biopsy forceps are simple in structure and convenient to assemble, so that biopsy forceps having an outer diameter of about 2 mm or less can be manufactured on the premise of ensuring structural strength and processing stability, and the biopsy forceps are suitable for passing through a small-sized lumen (e.g., nasal cavity or trachea) and performing a biopsy operation.

### BRIEF DESCRIPTION OF THE DRAWINGS

FIG. 1 is a schematic view showing a closed state of biopsy forceps according to a first embodiment of the present disclosure.
FIG. 2 is a schematic cross-sectional state view of the biopsy forceps shown in FIG. 1.
FIG. 3 is a schematic view showing an open state of biopsy forceps according to the first embodiment of the present disclosure.
FIG. 4 is a schematic cross-sectional state view of the biopsy forceps shown in FIG. 3.
FIG. 5 is a schematic structural view of a jaw base used in the first embodiment.
FIG. 6 is a schematic structural view of a square pin used in the first embodiment.
FIG. 7 is a schematic structural view of a sampling needle used in the first embodiment.
FIG. 8 is a schematic view showing a fixed state of the square pin shown in FIG. 6 and the sampling needle shown in FIG. 7.
FIG. 9 is a schematic structural view of a jaw used in the first embodiment.
FIG. 10 is a schematic structural view of biopsy forceps according to a non-claimed example of the present disclosure.
FIG. 11 is a schematic view showing a connection of two jaws and a pulling member in the biopsy forceps according to the non-claimed example
FIG. 12 is a schematic structural view of a jaw base used in the non-claimed example
FIG. 13 is a schematic structural view of a jaw used in the non-claimed example
FIG. 14 is a schematic cross-sectional structural view of a pulling member used in the non-claimed example.

### DETAILED DESCRIPTION

The technical solutions of the present disclosure are further described in detail below with reference to the accompanying drawings and specific embodiments.

Embodiments of the present disclosure provide improved biopsy forceps. By simplifying the structure of a jaw assembly, parts of the biopsy forceps are reduced, the assembly is simplified, and an overall size of the biopsy forceps is reduced, so that the biopsy forceps can pass through a small-sized lumen (e.g., nasal cavity) and perform a biopsy operation. In the following description, a side of the biopsy forceps near a lesion is a front end, which is also referred to as a distal end, and a side of the biopsy forceps near an operating handle is a rear end, which is also referred to as a proximal end.

### First Embodiment

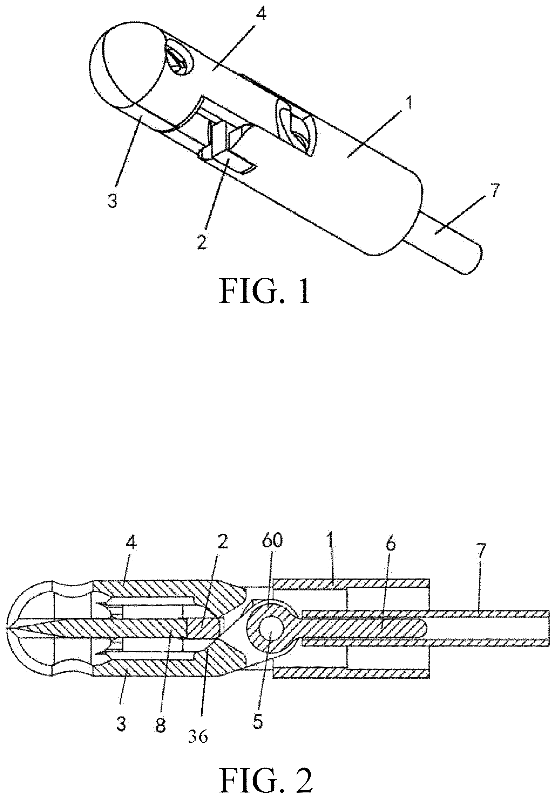

As shown in FIG. 1 to FIG. 4, the jaw assembly of biopsy forceps provided by the present embodiment includes a jaw base 1, a limiting element 2, a first jaw 3, a second jaw 4, a shaft member 5, and a pulling member 9.

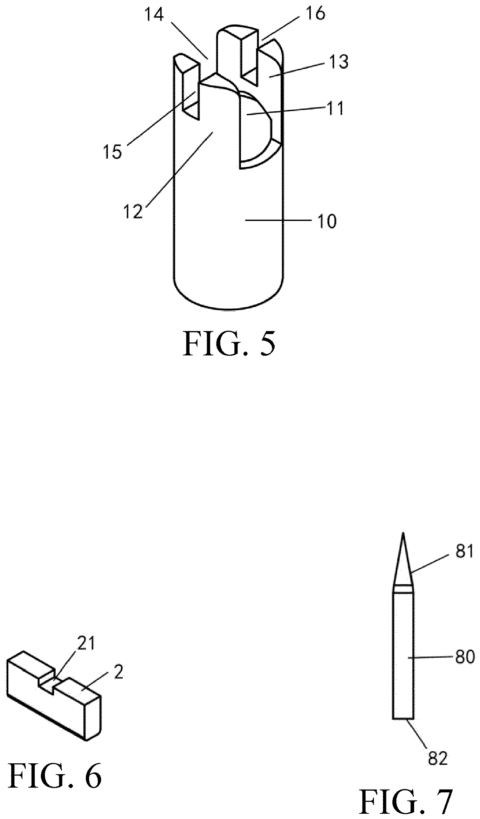

As shown in FIG. 5, the jaw base 1 includes a hollow tube 10, a first arm 12, and a second arm 13, which are integrally formed. A hollow cavity 11 is formed inside the hollow tube 10. An outer diameter of the hollow tube 10 may be 2.0-2.5 mm (e.g., 2.35-2.45 mm) or may be sized differently depending on different specifications of biopsy forceps. An outer diameter size of the hollow tube 10 serves as an outer diameter size of the jaw base 1 and is further the same as an outer diameter size of the jaw assembly. An axis of the hollow tube 10 is consistent with an axis of the jaw assembly. The first arm 12 and the second arm 13 are oppositely arranged at a front end of the hollow tube 10. An elongated slot 14 for accommodating the first jaw 3 and the second jaw 4 is formed between the first arm 12 and the second arm 13. A length direction of the elongated slot 14 is consistent with an axial direction of the hollow tube 10.

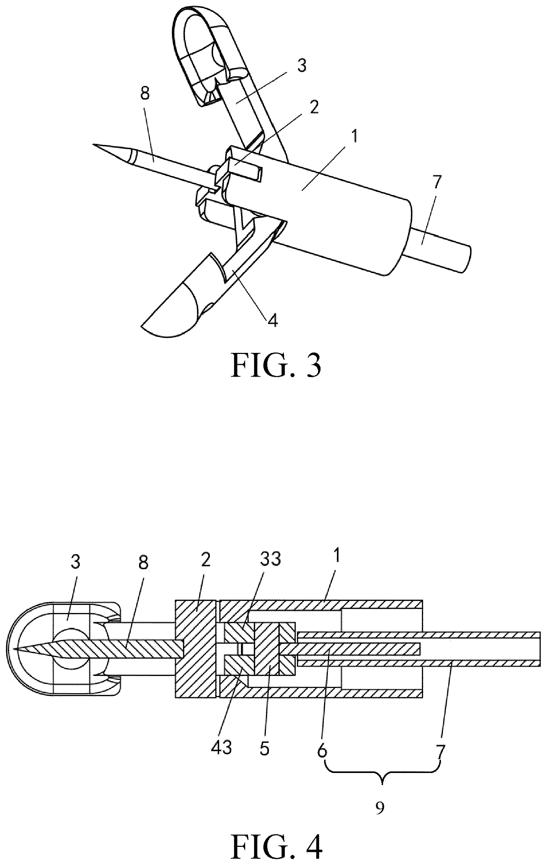

The first jaw 3 and the second jaw 4 are oppositely arranged, and a rear end 33 of the first jaw 3 and a rear end 43 of the second jaw 4 are arranged in the elongated slot 14 of the jaw base 1 (in combination with FIG. 1 and FIG. 4). Moreover, the first jaw 3 and the second jaw 4 may move along the elongated slot 14 of the jaw base 1. The rear end 33 of the first jaw 3, the rear end 43 of the second jaw 4, and a front end of the pulling member 9 are riveted by a shaft member 5 (e.g., rivet) (referring to FIG. 4). The pulling member 9 extends along the cavity 11 of the hollow tube 10.

The rear ends of the first jaw and the second jaw are further provided with ramps. The ramps are located at a front end of the shaft member and on both sides of the limiting element. The two ramps on the first jaw and the second jaw abut against both sides of the limiting element respectively, and may slide relative to the limiting element. The opposite surfaces of the ramps are each in the shape of a bevel that gradually protrudes toward the axis of the jaw assembly in a direction from the front end to the rear end (i.e., from the jaw spoon to the connecting portion). In other words, a distance between the ramp 36 on the first jaw 3 and the ramp 36 on the second jaw 4 is gradually reduced from the front end to the rear end. In the present embodiment, in particular in an example shown in FIG. 2, a minimum distance between the two ramps 36 is smaller than a size of the limiting element 2 between the two ramps 36, and the minimum distance between the two ramps 36 is between the limiting element 2 and the shaft member 5.

The limiting element 2 is arranged between the first jaw 3 and the second jaw 4. Both ends of the limiting element 2 are fixed to the first arm 12 and the second arm 13 respectively. The limiting element 2 is configured to limit a maximum stroke of the pulling member 9 pushing forward.

As shown in FIG. 1 and FIG. 2, the rear end 33 of the first jaw 3 and the rear end 43 of the second jaw 4 move toward the rear end (proximal end) in the elongated slot 14 of the jaw base 2 by pulling the pulling member 9 backward. At this moment, the limiting element 2 is matched with the ramps 36 to force the front ends of the first jaw 3 and the second jaw 4 to be closed. Meanwhile, during the closing process of the first jaw 3 and the second jaw 4, the first arm 12 and the second arm 13 on both sides of the elongated slot 14 also have a limiting effect on handles of the first jaw 3 and the second jaw 4, thereby enhancing the stability of the two jaws. The rear end 33 of the first jaw 3 and the rear end 43 of the second jaw 4 move toward the front end in the elongated slot 14 of the jaw base 2 by pushing the pulling member 9 forward. At this moment, the limiting element 2 is matched with the ramps 36 to force the front ends of the first jaw 3 and the second jaw 4 to be opened.

Specifically, when the pulling member 9 moves forward relative to the first arm 12 and the second arm 13, the first jaw 3 and the second jaw 4 are pushed forward along the elongated slot 14. Since the limiting element 2 is fixed to the first arm 12 and the second arm 13 and the ramps 36 are fixed to the first jaw 3 and the second jaw 4, the ramps 36 move forward relative to the limiting element 2. Since a space between the ramps 36 on the first jaw 3 and the second jaw 4 has a large front end and a small rear end, the distance between the ramp 36 on the first jaw 3 and the ramp 36 on the second jaw 4 is decreasing as the two ramps move toward the front end relative to the limiting element 2, and the limiting element 2 abuts against the surfaces of the ramp 36 on the first jaw 3 and the ramp 36 on the second jaw 4 to force the two ramps 36 to relatively move in a direction away from an axis of the jaw assembly, thereby driving the first jaw 3 and the second jaw 4 away from the axis of the jaw assembly (to be opened).

Similarly, when the pulling member 9 is pulled toward the rear end, the distance between the ramp 36 on the first jaw 3 and the ramp 36 on the second jaw 4 is increasing as the two ramps move toward the rear end relative to the limiting element 2, so that the first jaw 3 and the second jaw 4 are restored to a closed state.

As shown in FIG. 3 and FIG. 4, the front ends of the first jaw 3 and the second jaw 4 are opened under the action of the limiting element 2 by pushing the pulling member forward. When the front end of the pulling member 9 abuts against the rear end of the limiting element 2, the limiting element 2 prevents the pulling member from moving forward, and the pulling member stops pushing forward. An extreme position of the pulling member pulling backward is shown in FIG. 2. An extreme position of the pulling member pushing forward is shown in FIG. 4. It can also be seen from the figure that in small-sized biopsy forceps, a moving distance of the front end of the pulling member is short, and an operating space is narrow.

As shown in FIG. 2 to FIG. 4, the present embodiment provides needle biopsy forceps. The jaw assembly further includes a sampling needle 8. A rear end of the sampling needle 8 is fixed to the middle of the limiting element 2. By arranging the sampling needle 8 between the first jaw 3 and the second jaw 4, continuous multiple sampling can be achieved, and a sample may be stored on the entire sampling needle 8.

As shown in FIG. 5, in the jaw base 1 provided by the present embodiment, a front end of the first arm 12 is provided with a first fixing slot 15. A front end of the second arm 13 is provided with a second fixing slot 16. The first fixing slot 15 and the second fixing slot 16 are oppositely arranged. A length direction of the first fixing slot 15 and the second fixing slot 16 is consistent with an axial direction of the hollow tube 10. The first fixing slot 15 and the second fixing slot 16 have depth less than that of the elongated slot 14. Both ends of the limiting element 2 are fixed to the first fixing slot 15 and the second fixing slot 16 respectively, so that the limiting element 2 is fixed to the front ends of the first arm 12 and the second arm 13.

In order to facilitate the fixing of the limiting element 2 and the sampling needle 8, the cross sections of the first fixing slot 15 and the second fixing slot 16 are designed to be rectangular, and the cross section of the limiting element 2 is designed to be rectangular. Both ends of the limiting element 2 are located in the first fixing slot 15 and the second fixing slot 16, respectively, and are fixed by welding.

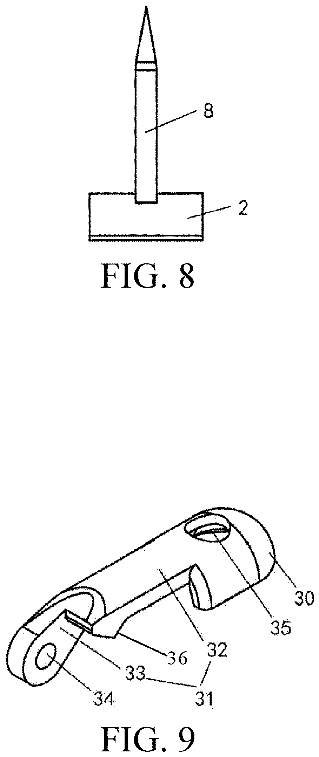

As shown in FIG. 6 and FIG. 8, the middle of the limiting element 2 is provided with a third fixing slot 21, and an opening of the third fixing slot 21 faces forward, so that a large surface is formed at the bottom of the third fixing slot 21, and the fixing of the sampling needle 8 is facilitated. A rear end 82 of the sampling needle 8 is fixed in the third fixing slot 21.

The structure of the sampling needle 8 is as shown in FIG. 7. The sampling needle 8 includes a cylindrical needle body 80 and a conical needle point 81. The bottom (the rear end 82 of the sampling needle) of the cylindrical needle body 80 is fixed into the third fixing slot 21 (referring to FIG. 8). In the present embodiment, the needle-type sampling needle 8 is used to facilitate penetration and sample storage in a biopsy operation, and continuous multiple sampling can be performed at the same lesion without the operation of repeatedly advancing and retracting the first and second jaws.

The structure of the first jaw 3 used in the present embodiment is as shown in FIG. 9, and the structure of the second jaw 4 is symmetrical to the structure of the first jaw 3. The first jaw 3 and the second jaw 4 both adopt an elongated handle.

As shown in FIG. 9, the first jaw 3 comprises a jaw spoon 30 and a handle 31. The jaw spoon 30 is located at a front end of the first jaw 3. The jaw spoon 30 is connected to a front end of the handle 31. The jaw spoon 30 and the handle 31 are integrally formed. Preferably, the first jaw 3 and the second jaw 4 are manufactured by powder metallurgy. Preferably, a window 35 may be provided in the jaw spoon 30, and tissue stored on the sampling needle 8 may be observed through the window 35 to facilitate the biopsy operation.

The description is made by taking an opening direction of the jaw spoon 30 as an inner side, an axial direction of the elongated handle 31 as a length direction, and a direction perpendicular to the length direction of the handle 31 as a width direction. The handle 31 includes a handle body 32 in the middle of the first jaw 3 and a connecting portion 33 at a rear end of the first jaw 3. The handle body 32 is configured to connect the jaw spoon 30 and the connecting portion 33. The connecting portion 33 is provided with a connecting hole 34 for realizing riveting of the first jaw 3 and the pulling member.

The jaw spoon 30 is wider than the handle body 32, and a center line of the handle body 32 coincides with a center line of the jaw spoon 30. The handle body 32 has the same arc-shaped cross section in the length direction. The arc-shaped cross section faces the opening direction of the jaw spoon 30, i.e. the inner side of the first jaw 3. Preferably, the handle body 32 has a semicircular cross section. The handle body 32 protrudes outwardly of the jaw spoon 30 to form an inwardly facing groove in which a sample may be stored. By increasing the length of the handle body 32, the storage space for the sample may be increased. Certainly, the handle body 32 should not be too long in view of the moving space of the small-sized biopsy forceps.

The handle body 32 is wider than the connecting portion 33. The connecting portion 33 is arranged away from the axis of the handle body 32. In the width direction, the connecting portion 33 is biased toward one side of the handle body 32. Thus, when the jaw spoons 30 of the first jaw 3 and the second jaw 4 are oppositely arranged, the connecting holes in the rear ends of the first jaw 3 and the second jaw 4 may be directly riveted with the front end of the pulling member through the shaft member 5. On one hand, the first jaw 3 and the second jaw 4 are pulled or pushed to move integrally relative to the first arm or the second arm. On the other hand, the first jaw 3 and the second jaw 4 are opened or combined by using the limiting element, so that the first jaw 3 and the second jaw 4 are integrally advanced and retracted, and the two jaw spoons 30 and the handle body 32 are opened and closed.

As shown in FIG. 9, the connecting portion 33 is equal in width and protrudes toward the inner side of the handle body 32, and a protruding direction of the connecting portion 33 from the handle body 32 is the same as the opening direction of the jaw spoon 31 (toward the jaw spoon of the second jaw 4). The connecting portion 33 is provided with a connecting hole 34, and an axis of the connecting hole 34 is perpendicular to a length direction of the first jaw 3. The first jaw 3, the second jaw 4, and the pulling member are riveted through the shaft member 5. Therefore, the connecting portion and the pulling member may move toward the front end or the rear end together, and the first jaw 3 and the second jaw 4 may rotate around the shaft member 5 to open and close the jaw spoon. In the present embodiment, the connecting portion 33 has a circular arc-shaped outer contour around the connecting hole 34, so that the structural strength and the processing stability of small-sized jaws can be ensured.

In the jaw assembly provided by the present embodiment, by improving the structures of the first jaw 3 and the second jaw 4, a pin hole provided in the middle of the handle body is omitted, the handle body is provided as a groove capable of accommodating a sample, and the first jaw 3 and the second jaw 4 are riveted through the connecting hole at the rear end. The first jaw 3 and the second jaw 4 rotate around the shaft member at the rear end, so that the sample may be stored on the sampling needle 8 between the jaw spoons 31 and the handles 32 of the two jaws, and is not limited by the structures of the jaws. In conventional biopsy forceps, a four-linkage structure is used for driving, and the jaws rotate around pins at handle portions, so that the sample can be stored only between the two jaw spoons. Compared with the conventional biopsy forceps, the above biopsy forceps obviously increase the storage space of samples. Thus, the overall size of the jaw assembly is reduced, the sample storage space of the jaw portions is ensured, and the requirement of continuous multiple sampling of small-sized biopsy forceps is met.

In addition, in the above jaw structure, a hole in the middle of the handle body in the four-linkage structure in the conventional biopsy forceps is omitted, and the connecting hole is provided at a central shaft position of the connecting portion at the rear ends of the first jaw 3 and the second jaw 4, so that the jaw spoon 31, the handle body 32, and the connecting portion 33 are integrally formed on a straight line and have a rigid structure, the mechanical strength of the jaws is effectively improved, and therefore the jaws can be prevented from being broken during manufacture and use. Moreover, since the overall size of the jaw assembly is reduced, only the pulling member is used for pushing and pulling the rear ends of the first jaw 3 and the second jaw 4, an acting force required for forcing the two jaws to move can be provided, and thus the use requirement of a small-sized jaw base is met.

Referring to FIG. 2 and FIG. 4, the pulling member 9 used in the present embodiment includes a pull rod 6 and a connecting tube 7. The pull rod 6 and the connecting tube 7 are short. An annular connecting portion 60 is arranged at a front end of the cylindrical pull rod 6, and the shaft member 5 penetrates through the annular connecting portion 60 to realize riveting of the pulling member with the first jaw 3 and the second jaw 4. The cylindrical pull rod 6 is inserted into a front end of the connecting tube 7 and then fixed to form the pulling member.

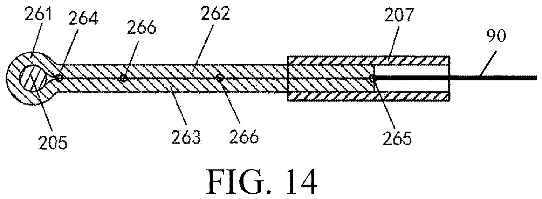

Biopsy forceps manufactured using the jaw assembly provided by the present embodiment include the above jaw assembly, a controlling wire, a spring tube, and an operating handle. A rear end of the connecting tube 7 is fixed to a front end of the controlling wire 90 (referring to FIG. 14), and a rear end of the controlling wire 90 is fixed to the operating handle. The spring tube is sleeved over the controlling wire 90. A front end of the spring tube is fixed to a rear end of the jaw base 1, and a rear end of the spring tube is fixed to the operating handle to form the biopsy forceps.

According to the above biopsy forceps, the structure of the jaw assembly is simplified, so that the size of the entire jaw portions can be reduced, the production of the biopsy forceps used under a nasal endoscope can be effectively realized, and the problems that an existing biopsy forceps product is complex to assemble, small in part size and unstable in strength reliability are solved. Some parts are reduced through reasonable structural design, the parts of the small-sized biopsy forceps can be optimized, the assembly difficulty can be maximally reduced, and the production efficiency can be improved. Moreover, by improving the structures of the jaws, a sample storage space which cannot be provided by the existing biopsy forceps is provided, so that a continuous sampling function can be realized.

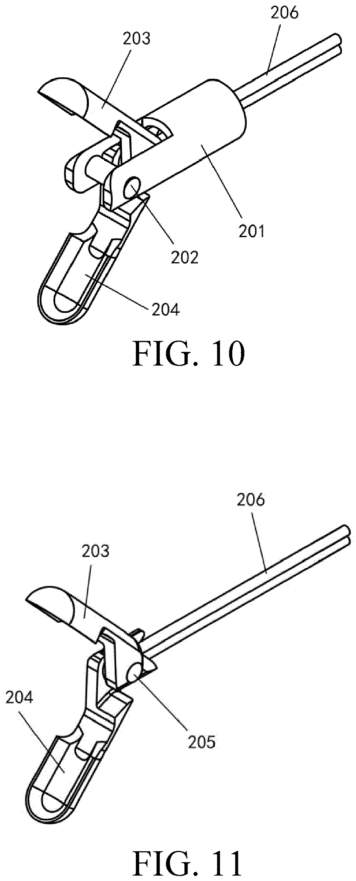

Non-claimed example As shown in FIG. 10 and FIG. 11, the jaw assembly of biopsy forceps provided by the present embodiment includes a jaw base 201, a limiting element 202, a first jaw 203, a second jaw 204, a shaft member 205, and a pulling member.

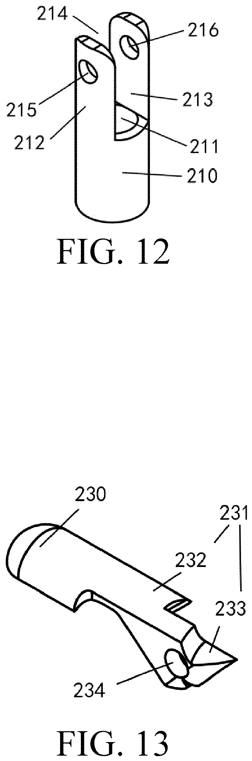

As shown in FIG. 12, the jaw base 201 includes a hollow tube 210, a first arm 212, and a second arm 213, which are integrally formed. The hollow tube 210 defines a hollow cavity 211. An outer diameter of the hollow tube 210 may be controlled to be 0.8-1.0 mm or may be sized differently depending on different specifications of biopsy forceps. An outer diameter size of the hollow tube 210 is an outer diameter size of the jaw base 201. An axis of the hollow tube 210 is consistent with an axis of the biopsy forceps. The first arm 212 and the second arm 213 are oppositely arranged at a front end of the hollow tube 210. An elongated slot 214 for accommodating rear ends of the first jaw 203 and the second jaw 204 is provided between the first arm 212 and the second arm 213. A length direction of the elongated slot 214 is consistent with an axial direction of the hollow tube 210.

The rear ends of the first jaw 203 and the second jaw 204 are oppositely arranged in the elongated slot 214 of the jaw base 201. Moreover, the first jaw 203 and the second jaw 204 may move in the elongated slot 214 of the jaw base 201. The rear end of the first jaw 203, the rear end of the second jaw 204, and a front end of the pulling member are riveted by the shaft member 205. The pulling member extends rearward through the cavity 211 of the hollow tube 210.

As shown in FIG. 10, the limiting element 202 is arranged between the first jaw 203 and the second jaw 204. Both ends of the limiting element 202 are fixed to the first arm 212 and the second arm 213 respectively. The limiting element 202 is configured to define a maximum position of the pulling member pushing forward, and the front ends of the first jaw 203 and the second jaw 204 are opened under the action of the limiting element 202 by pushing the pulling member forward. The first jaw 203 and the second jaw 204 are closed by pulling the pulling member backward, as defined by the elongated slot 214 of the jaw base 202.

As shown in FIG. 12, in the jaw base provided by the present embodiment, a first circular hole 215 is formed in the first arm 212, a second circular hole 216 is formed in the second arm 213, and both ends of the cylindrical limiting element 202 are fixed in the first circular hole 215 and the second circular hole 216, respectively.

The non-claimed example provides a pair of needleless biopsy forceps. Biopsy forceps of a smaller size can be achieved by reducing the size of parts in the jaw assembly. An outer diameter of the jaw base may be controlled to be about 1 mm, and the sizes of the first jaw 203, the second jaw 204, the limiting element 202, and the shaft member 205 are correspondingly reduced.

The structure of the first jaw 203 used in the non-claimed example is as shown in FIG. 13, and the structure of the second jaw 204 is the same as the structure of the first jaw 203. The first jaw 203 and the second jaw 204 both adopt an elongated handle. The structure of the jaws used in the non-claimed example is similar to that of the jaws used in the first embodiment, except that the jaws are smaller in size and can only be used for single sampling. There are also some differences in the structures of the jaws used in the non-claimed example and the jaws used in the first embodiment in order to meet the use requirement of smaller sizes.

As shown in FIG. 13, the first jaw 203 comprises a jaw spoon 230 and a handle 231. The jaw spoon 230 is located at a front end of the first jaw 203. The jaw spoon 230 is connected to a front end of the handle 231. The jaw spoon 230 and the handle 231 are integrally formed.

The description is made by taking an opening direction of the jaw spoon 230 as an inner side, an axial direction of the handle 231 as a length direction, and a direction perpendicular to the length direction of the handle 231 as a width direction. The handle 231 includes a handle body 232 in the middle of the first jaw 203 and a connecting portion 233 at a rear end of the first jaw 203. The handle body 232 refers to an arc-shaped portion of equal width and equal thickness in the handle 231. The handle body 232 is configured to connect the jaw spoon 230 and the connecting portion 233. The connecting portion 233 is provided with a connecting hole 234 for realizing riveting of the first jaw 203 and the pulling member.

The jaw spoon 230 is wider than the handle body 232, and a center line of the handle body 232 coincides with a center line of the jaw spoon 230. The handle body 232 has the same arc-shaped cross section in the length direction. The arc-shaped cross section faces the opening direction of the jaw spoon 230, i.e. the inner side of the first jaw 203. Preferably, the handle body 232 has a semicircular cross section. The handle body 232 protrudes outwardly of the jaw spoon 230 to form an inwardly facing groove in which a sample may be stored. By increasing the length of the handle body 232 in the present embodiment, the storage space for the sample can be increased. Certainly, the handle body 232 should not be too long in view of the moving space of the small-sized biopsy forceps.

The handle body 232 is wider than the connecting portion 233. The connecting portion 233 is arranged away from the axis of the handle body 232. In the width direction, the connecting portion 233 is biased toward one side of the handle body 232. Thus, when the jaw spoons 230 of the first jaw 203 and the second jaw 204 are oppositely arranged, the alignment and closure of the two jaw spoons and the handle body can be achieved by riveting the connecting portions 233 at the rear ends of the first jaw 203 and the second jaw 204 with the front end of the pulling member.

As shown in FIG. 13, the connecting portion 233 protrudes toward the inner side of the handle body 232, and a protruding direction of the connecting portion 233 is the same as the opening direction of the jaw spoon 231. The connecting portion 233 is provided with a connecting hole 234, and an axis of the connecting hole 234 is perpendicular to a length direction of the first jaw 203. Therefore, when the two connecting portions 233 of the first jaw 203 and the second jaw 204 are riveted with the pulling member through the shaft member 205, the first jaw 203 and the second jaw 204 may rotate around the shaft member 205 to open and close the jaw spoon. Since the sizes of the first jaw 203 and the second jaw 204 are small and the size of the connecting portion 233 is small, the size of the connecting hole 234 in the connecting portion 233 is also greatly reduced, thereby ensuring the structural stability of the jaws.

In the non-claimed example, in order that the first j aw 203 and the second jaw 204 may fit the small-sized jaw base 201, a contour of the connecting portion 233 near the inner side has an inclined structure. That is, opposite portions of the rear end of the first jaw 203 and the rear end of the second jaw 204 have an inclined contour. The moving space required for the connecting portion 233 provided with the inclined contour is also reduced during the rotation of the first jaw 203 and the second jaw 204 with respect to the structure in which the entire contour is circular arc-shaped.

Referring to FIG. 14, the pulling member used in the non-claimed example includes a filament 206 and a connecting tube 207 in order to fit the small-sized jaw base and jaws and to meet the operational stability of the pulling member. The filament 206 has a diameter ranging from 0.19 to 0.2 mm. The filament 206 is folded in half, an annular connecting portion 261 is formed in the middle of the filament 206, and the filament 206 is connected to the shaft member 205 through the annular connecting portion 261. The filament 206 has a plurality of connecting points between portions 262 and 263 extending from the middle to both ends. Preferably, a connecting point 264 is arranged at a position of the filament 206 near the annular connecting portion 261. Connecting points 265 are arranged at both ends of the filament 206, and a plurality of connecting points 266 are arranged between the connecting point 264 and the connecting points 265.

In the pulling member provided by the non-claimed example, an end portion of the filament 206 is inserted to one end of the connecting tube 207 to be fixed, and then the pulling member can be fixed to the controlling wire and the operating handle through the connecting tube 207.

In the non-claimed example, by pulling the two jaws using the filament 206 folded in half, not only is the size of the pulling member reduced, but also the strength of the pulling action is ensured. The length of the filament 206 after being folded in half is not less than 60 mm (e.g., 100 mm), and by arranging the connecting tube 207 having a larger diameter at a position far away from the jaws, it is possible to ensure that a portion having a small outer diameter of the biopsy forceps has a large length, thereby facilitating the insertion and movement of the biopsy forceps into a small-sized lumen.

Biopsy forceps manufactured using the jaw assembly provided by the non-claimed example include the above jaw assembly, the controlling wire, the spring tube, and the operating handle. A rear end of the connecting tube 207 is fixed to a front end of the controlling wire (not shown), and a rear end of the controlling wire is fixed to the operating handle. The spring tube is sleeved over the controlling wire. A front end of the spring tube is fixed to a rear end of the jaw base 201, and a rear end of the spring tube is fixed to the operating handle to form the biopsy forceps.

The biopsy forceps provided by the non-claimed example may be of a smaller size to fit a lumen of a smaller size (e.g., nasal cavity) than the biopsy forceps provided by the first embodiment.

In summary, in the biopsy forceps provided by the present disclosure, a four-linkage structure is removed from the jaw assembly, the rear ends of the first jaw and the second jaw are pushed and pulled only through the pulling member, and the first jaw and the second jaw are opened and closed by in combination with the limiting effects of the limiting element and the jaw base. The biopsy forceps are simple in structure and convenient to assemble, so that small-sized biopsy forceps can be achieved to be suitable for passing through a small-sized lumen and performing a biopsy operation.

In the descriptions of this specification, descriptions of reference terms such as "an embodiment," "some embodiments," "an example," "a specific example," or "some examples" mean that exemplary sizes, shapes, structures, materials, or features are included in at least one embodiment or example of this specification, and may be combined in a proper manner in any one or more of the embodiments or examples. The exemplary descriptions do not constitute a limitation to the present disclosure. In addition, a person skilled in the art may integrate or combine different embodiments or examples described in this specification and features of the different embodiments or examples as long as they are not contradictory to each other. The scope of the invention is defined by the appended claims solely.

In addition, terms "first" and "second" are used merely for the purpose of description, and shall not be construed as indicating or implying relative importance or implying a quantity of indicated technical features. Therefore, features defining "first" and "second" may explicitly or implicitly include at least one of the features. In descriptions of the present utility model, "a plurality of" means two or more, unless otherwise defined clearly and specifically.

The jaw assembly of biopsy forceps and biopsy forceps provided in the present disclosure are described in detail above.

## Claims

1. A jaw assembly of biopsy forceps, comprising: a jaw base (1), a first jaw (3), a second jaw (4), a limiting element (2), a pulling member (9), and a shaft member (5),
the jaw base comprising a hollow tube (10), a first arm (12), and a second arm (13), which are integrally formed, the first arm and the second arm standing oppositely at a front end of the hollow tube, and an elongated slot (14) between the first arm and the second arm for accommodating the first jaw and the second jaw, the pulling member extending along a cavity (11) of the hollow tube;
the first jaw and the second jaw being openable and closeable, and are movably arranged in the elongated slot of the jaw base;
a rear end (33) of the first jaw and a rear end (43) of the second jaw being relatively rotatably connected with a front end of the pulling member, and the pulling member extends rearward within the cavity (11) of the hollow tube,
wherein
the jaw assembly of biopsy forceps further includes a sampling needle (8),
the limiting element is arranged between the first jaw and the second jaw, two ends of the limiting element are respectively fixed to the first arm and the second arm, and the limiting element is configured to limit a maximum stroke of the pulling member pushing forward;
the first jaw and the second jaw are individually provided with a ramp for being matched with the limiting element to open or close the first jaw and the second jaw, the rear end (33) of the first jaw, the rear end (43) of the second jaw, and a front end of the pulling member are riveted by the shaft member; and
the pulling member includes a pull rod (6), an annular connecting portion (60) is arranged at a front end of the pull rod, and the shaft member penetrates through the annular connecting portion to realize riveting of the pulling member with the first jaw and the second jaw,
**characterized in that**:
a rear end of the sampling needle (8) is fixed to the middle of the limiting element (2), and the pulling member (9) further includes a connecting tube (7), the pull rod is inserted into a front end of the connecting tube.

2. The jaw assembly of biopsy forceps according to claim 1, wherein
a distance between the ramp of the first jaw and the ramp of the second jaw is gradually reduced to the rear end of the first jaw and the rear end of the second jaw.

3. The jaw assembly of biopsy forceps according to claim 2, wherein
the ramp of the first jaw or the second jaw is located in front of the shaft member.

4. The jaw assembly of biopsy forceps according to claim 2, wherein
a minimum distance between the ramp of the first jaw and the ramp of the second jaw is between the limiting element and the shaft member.

5. The jaw assembly of biopsy forceps according to claim 4, wherein
the minimum distance is smaller than a size of the limiting element located between the ramp of the first jaw and the ramp of the second jaw.

6. The jaw assembly of biopsy forceps according to claim 4, wherein
the first arm is provided with a first fixing slot (15), the second arm is provided with a second fixing slot (16), both ends of the limiting element are fixed to the first fixing slot and the second fixing slot respectively, and the first fixing slot and the second fixing slot are less than the elongated slot in depth.

7. The jaw assembly of biopsy forceps according to claim 2, wherein
the first jaw and the second jaw individually comprise a jaw spoon (30) and a handle (31), which are integrally formed, the handle comprises a handle body (32) and a connecting portion (33), and the handle body is configured to connect the jaw spoon and the connecting portion; and
the first jaw and the second jaw are relatively rotatably connected at the connecting portion.

8. The jaw assembly of biopsy forceps according to claim 7, wherein
the jaw spoon, the handle, and the connecting portion are integrally formed along a straight line to form a rigid structure.

9. The jaw assembly of biopsy forceps according to claim 7, wherein
the connecting portion connects the first jaw and the second jaw and is connected to the pulling member, and the connecting portion and the pulling member are configured to together move forward or rearward relative to the jaw base.

10. The jaw assembly of biopsy forceps according to claim 1, wherein
the rear end of the first jaw and the rear end of the second jaw have opposite portions in inclined contour.

11. A biopsy forceps, comprising the jaw assembly according to any one of claims 1-10, and further comprising: a spring tube, a controlling wire, and an operating handle, wherein a front end of the spring tube is fixed to a rear end of the jaw base, a rear end of the spring tube is fixed to the operating handle, a rear end of the pulling member is fixed to a front end of the controlling wire, the controlling wire extends rearward through the spring tube, and a rear end of the controlling wire is fixed to the operating handle.

## Patentansprüche

1. Backenanordnung einer Biopsiezange, umfassend: eine Backenbasis (1), eine erste Backe (3), eine zweite Backe (4), ein Begrenzungselement (2), ein Zugelement (9) und ein Schaftelement (5),
wobei die Backenbasis ein Hohlrohr (10), einen ersten Arm (12) und einen zweiten Arm (13) umfasst, die einstückig geformt sind, wobei der erste Arm und der zweite Arm gegenüberliegend an einem vorderen Ende des Hohlrohrs stehen, und einen länglichen Schlitz (14) zwischen dem ersten Arm und dem zweiten Arm zum Aufnehmen der ersten Backe und der zweiten Backe, wobei sich das Zugelement entlang eines Hohlraums (11) des Hohlrohrs erstreckt;
wobei die erste Backe und die zweite Backe geöffnet und geschlossen werden können und beweglich in dem länglichen Schlitz der Backenbasis angeordnet sind;
ein hinteres Ende (33) der ersten Backe und ein hinteres Ende (43) der zweiten Backe relativ drehbar mit einem vorderen Ende des Zugelements verbunden sind und das Zugelement sich innerhalb des Hohlraums (11) des Hohlrohrs nach hinten erstreckt,
wobei
die Backenbaugruppe der Biopsiezange weiter eine Probenahmenadel (8) beinhaltet,
das Begrenzungselement zwischen der ersten Backe und der zweiten Backe angeordnet ist, zwei Enden des Begrenzungselements jeweils am ersten Arm und am zweiten Arm befestigt sind, und das Begrenzungselement konfiguriert ist, einen maximalen Hub des nach vorne drückenden Zugelements zu begrenzen;
die erste Backe und die zweite Backe einzeln mit einer Rampe versehen sind, um mit dem Begrenzungselement zum Öffnen oder Schließen der ersten Backe und der zweiten Backe zusammenzupassen, wobei das hintere Ende (33) der ersten Backe, das hintere Ende (43) der zweiten Backe und ein vorderes Ende des Zugelements mit dem Schaftelement vernietet sind; und
das Zugelement eine Zugstange (6) beinhaltet, ein ringförmiger Verbindungsabschnitt (60) an einem vorderen Ende der Zugstange angeordnet ist und das Schaftelement durch den ringförmigen Verbindungsabschnitt dringt, um eine Vernietung des Zugelements mit der ersten Backe und der zweiten Backe zu realisieren,
**dadurch gekennzeichnet, dass**:
ein hinteres Ende der Probenahmenadel (8) in der Mitte des Begrenzungselements (2) befestigt ist und
das Zugelement (9) weiter ein Verbindungsrohr (7) beinhaltet, wobei die Zugstange in ein vorderes Ende des Verbindungsrohrs eingeführt ist.

2. Backenanordnung einer Biopsiezange nach Anspruch 1, wobei
ein Abstand zwischen der Rampe der ersten Backe und der Rampe der zweiten Backe zum hinteren Ende der ersten Backe und zum hinteren Ende der zweiten Backe hin allmählich verringert wird.

3. Backenanordnung einer Biopsiezange nach Anspruch 2, wobei
die Rampe der ersten Backe oder der zweiten Backe vor dem Schaftelement liegt.

4. Backenanordnung einer Biopsiezange nach Anspruch 2, wobei
zwischen dem Begrenzungselement und dem Schaftelement ein Mindestabstand zwischen der Rampe der ersten Backe und der Rampe der zweiten Backe besteht.

5. Backenanordnung einer Biopsiezange nach Anspruch 4, wobei
der Mindestabstand kleiner ist als eine Größe des Begrenzungselements, das sich zwischen der Rampe der ersten Backe und der Rampe der zweiten Backe befindet.

6. Backenanordnung einer Biopsiezange nach Anspruch 4, wobei
der erste Arm mit einem ersten Befestigungsschlitz (15) versehen ist, der zweite Arm mit einem zweiten Befestigungsschlitz (16) versehen ist, beide Enden des Begrenzungselements am ersten Befestigungsschlitz beziehungsweise am zweiten Befestigungsschlitz befestigt sind, und der erste Befestigungsschlitz und der zweite Befestigungsschlitz weniger tief als der längliche Schlitz sind.

7. Backenanordnung einer Biopsiezange nach Anspruch 2, wobei
die erste Backe und die zweite Backe einzeln einen Backenlöffel (30) und einen Griff (31) umfassen, die einstückig ausgebildet sind, der Griff einen Griffkörper (32) und einen Verbindungsabschnitt (33) umfasst und der Griffkörper konfiguriert ist, den Backenlöffel und den Verbindungsabschnitt zu verbinden; und
die erste Backe und die zweite Backe am Verbindungsabschnitt relativ drehbar verbunden sind.

8. Backenanordnung einer Biopsiezange nach Anspruch 7, wobei
der Backenlöffel, der Griff und der Verbindungsabschnitt einstückig entlang einer geraden Linie gebildet sind, um eine starre Struktur zu bilden.

9. Backenanordnung einer Biopsiezange nach Anspruch 7, wobei
der Verbindungsabschnitt die erste Backe und die zweite Backe verbindet und mit dem Zugelement verbunden ist und der Verbindungsabschnitt und das Zugelement konfiguriert sind, sich gemeinsam relativ zur Backenbasis vorwärts oder rückwärts zu bewegen.

10. Backenanordnung einer Biopsiezange nach Anspruch 1, wobei
das hintere Ende der ersten Backe und das hintere Ende der zweiten Backe gegenüberliegende Abschnitte mit geneigter Kontur aufweisen.

11. Biopsiezange, die die Backenanordnung nach einem der Ansprüche 1-10 umfasst und weiter umfasst: ein Federrohr, einen Steuerdraht und einen Betätigungsgriff, wobei ein vorderes Ende des Federrohrs an einem hinteren Ende der Backenbasis befestigt ist, ein hinteres Ende des Federrohrs am Betätigungsgriff befestigt ist, ein hinteres Ende des Zugelements an einem vorderen Ende des Steuerdrahts befestigt ist, der Steuerdraht sich nach hinten durch das Federrohr erstreckt und ein hinteres Ende des Steuerdrahts am Betätigungsgriff befestigt ist.

## Revendications

1. Ensemble mâchoire de pince de biopsie, comprenant : une base de mâchoire (1), une première mâchoire (3), une seconde mâchoire (4), un élément de limitation (2), un élément de traction (9) et un élément d'arbre (5),
la base de mâchoire comprenant un tube creux (10), un premier bras (12) et un second bras (13), qui sont formés d'un seul tenant, le premier bras et le second bras se trouvant de façon opposée au niveau d'une extrémité avant du tube creux, et une fente allongée (14) entre le premier bras et le second bras pour loger la première mâchoire et la seconde mâchoire, l'élément de traction s'étendant le long d'une cavité (11) du tube creux ;
la première mâchoire et la seconde mâchoire pouvant être ouvertes et fermées et étant agencées de manière mobile dans la fente allongée de la base de mâchoire ;
une extrémité arrière (33) de la première mâchoire et une extrémité arrière (43) de la seconde mâchoire étant raccordées de manière relativement rotative à une extrémité avant de l'élément de traction et l'élément de traction s'étend vers l'arrière à l'intérieur de la cavité (11) du tube creux,
dans lequel
l'ensemble mâchoire de pince de biopsie inclut en outre une aiguille d'échantillonnage (8),
l'élément de limitation est agencé entre la première mâchoire et la seconde mâchoire, deux extrémités de l'élément de limitation sont respectivement fixées au premier bras et au second bras et l'élément de limitation est configuré pour limiter une course maximale de l'élément de traction poussant vers l'avant ;
la première mâchoire et la seconde mâchoire sont individuellement pourvues d'une rampe pour être adaptées à l'élément de limitation pour ouvrir ou fermer la première mâchoire et la seconde mâchoire, l'extrémité arrière (33) de la première mâchoire, l'extrémité arrière (43) de la seconde mâchoire et une extrémité avant de l'élément de traction sont rivetées par l'élément d'arbre ; et
l'élément de traction inclut une tige de traction (6), une portion de raccordement annulaire (60) est agencée au niveau d'une extrémité avant de la tige de traction et l'élément d'arbre pénètre à travers la portion de raccordement annulaire pour réaliser le rivetage de l'élément de traction avec la première mâchoire et la seconde mâchoire,
**caractérisé en ce que** :
une extrémité arrière de l'aiguille d'échantillonnage (8) est fixée au milieu de l'élément de limitation (2), et
l'élément de traction (9) inclut en outre un tube de raccordement (7), la tige de traction est insérée dans une extrémité avant du tube de raccordement.

2. Ensemble mâchoire de pince de biopsie selon la revendication 1, dans lequel
une distance entre la rampe de la première mâchoire et la rampe de la seconde mâchoire est progressivement réduite jusqu'à l'extrémité arrière de la première mâchoire et l'extrémité arrière de la seconde mâchoire.

3. Ensemble mâchoire de pince de biopsie selon la revendication 2, dans lequel
la rampe de la première mâchoire ou de la seconde mâchoire est située devant l'élément d'arbre.

4. Ensemble mâchoire de pince de biopsie selon la revendication 2, dans lequel
une distance minimale entre la rampe de la première mâchoire et la rampe de la seconde mâchoire est entre l'élément de limitation et l'élément d'arbre.

5. Ensemble mâchoire de pince de biopsie selon la revendication 4, dans lequel
la distance minimale est inférieure à une taille de l'élément de limitation situé entre la rampe de la première mâchoire et la rampe de la seconde mâchoire.

6. Ensemble mâchoire de pince de biopsie selon la revendication 4, dans lequel
le premier bras est pourvu d'une première fente de fixation (15), le second bras est pourvu d'une seconde fente de fixation (16), les deux extrémités de l'élément de limitation sont respectivement fixées à la première fente de fixation et à la seconde fente de fixation et la première fente de fixation et la seconde fente de fixation présentent une profondeur inférieure à celle de la fente allongée.

7. Ensemble mâchoire de pince de biopsie selon la revendication 2, dans lequel
la première mâchoire et la seconde mâchoire comprennent individuellement une cuillère de mâchoire (30) et un manche (31), qui sont formés d'un seul tenant, le manche comprend un corps de manche (32) et une portion de raccordement (33) et le corps de manche est configuré pour raccorder la cuillère de mâchoire et la portion de raccordement ; et
la première mâchoire et la seconde mâchoire sont raccordées de manière relativement rotative au niveau de la portion de raccordement.

8. Ensemble mâchoire de pince de biopsie selon la revendication 7, dans lequel
la cuillère de mâchoire, le manche et la portion de raccordement sont formés d'un seul tenant le long d'une ligne droite pour former une structure rigide.

9. Ensemble mâchoire de pince de biopsie selon la revendication 7, dans lequel
la portion de raccordement raccorde la première mâchoire et la seconde mâchoire et est raccordée à l'élément de traction et la portion de raccordement et l'élément de traction sont configurés pour se déplacer ensemble vers l'avant ou vers l'arrière par rapport à la base de mâchoire.

10. Ensemble mâchoire de pince de biopsie selon la revendication 1, dans lequel
l'extrémité arrière de la première mâchoire et l'extrémité arrière de la seconde mâchoire présentent des portions opposées dans le contour incliné.

11. Pince de biopsie, comprenant l'ensemble mâchoire selon l'une quelconque des revendications 10, et comprenant en outre : un tube à ressort, un fil de commande et une poignée de commande, dans laquelle une extrémité avant du tube à ressort est fixée à une extrémité arrière de la base de mâchoire, une extrémité arrière du tube à ressort est fixée à la poignée de commande, une extrémité arrière de l'élément de traction est fixée à une extrémité avant du fil de commande, le fil de commande s'étend vers l'arrière à travers le tube à ressort et une extrémité arrière du fil de commande est fixée à la poignée de commande.
